# EUROPÄISCHE PATENTSCHRIFT

(11) **EP 1 060 762 B1**
(45) Veröffentlichungstag und Bekanntmachung des Hinweises auf die Patenterteilung: **30.08.2006**
(21) Anmeldenummer: 00108600.8
(22) Anmeldetag: 20.04.2000
(51) Int. Cl.: A61N 1/37, H04B 15/00, A61N 1/39

(54) **Verfahren und Vorrichtung zur Unterscheidung zwischen elektromagnetischen Störsignalen und elektromedizinischen Abtastsignalen insbesondere von kardiologischen Implantaten**
A method and apparatus for discriminating between electromagnetic interference signals and electromedical sample signals, in particular from cardiological implants
Procédé et appareil pour discriminer entre des signaux d'interférences électromagnétiques et des signaux d'échantillonnages électromédicaux, en particulier venant d'implants cardiologiques

(30) Priorität: 17.06.1999 DE 19927616
(43) Veröffentlichungstag der Anmeldung: 20.12.2000
(73) Patentinhaber: BIOTRONIK GmbH & Co. KG, 12359 Berlin (DE)
(72) Erfinder: Nappholz, Tibor, Evergreen, CO 80439 (US)
(74) Vertreter: Lindner-Vogt, Karin L.

(56) Entgegenhaltungen:
- US-A- 4 531 526
- US-A- 5 292 348

## Beschreibung

Die Erfindung betrifft ein Verfahren und eine Vorrichtung zur Unterscheidung zwischen extern eingestrahlten elektromagnetischen Störsignalen und elektromedizinischen Abtastsignalen, insbesondere bei Eingangssignalen von kardiologischen Implantaten, wie Herzschrittmachern, Defibrillatoren und dergleichen, bei neurologischen Reizdetektionen usw.

Die der Erfindung zugrunde liegende Problematik liegt in der zunehmenden "Verseuchung" unseres Lebensraumes mit elektromagnetischen Strahlungen unterschiedlichster Herkunft. Jedes elektronische Gerät und insbesondere auch kardiologische Implantate sind einer Vielzahl verschiedener elektromagnetischer Umgebungsstrahlungen (EMI = Environmental Electromagnetic Interference) ausgesetzt. Dieses Problem wurde erheblich dadurch verschärft, daß einerseits EMI-Signalquellen, wie z.B. Alarmanlagen, Anti-Diebstahl-Anlagen, Mobilfunktelefone usw., dramatisch zugenommen haben und andererseits moderne elektromedizinische Geräte immer empfindlicher werden, also z.B. moderne kardiologische Implantate extrem kleine Signale erfassen und auswerten, um den tatsächlichen Zustand des Herzens möglichst genau zu diagnostizieren und darauf basierend möglichst optimale therapeutische Impulse für das funktionsgestörte Herz zu erzeugen. Eine schlichte Abschirmung des Implantates durch ein MetallGehäuse ist dabei nicht ausreichend, da über die angeschlossenen Signalleitungen und deren Eingänge am Implantat Störsignale eingestreut werden können.

Zur Abhilfe ist es aus dem Stand der Technik bekannt, spezielle Durchführungen für die Anschlüsse des Implantates vorzusehen, die allerdings nur für sehr hohe Frequenzen, wie sie z.B. bei Mobilfunktelefonen eingesetzt werden, ausreichend wirksam sind. Niedrigere Frequenzen, wie sie z.B. von Alarm- bzw. Anti-Diebstahl-Anlagen erzeugt werden, Netzfrequenzen und andere industrielle Störsignalquellen sind jedoch weniger gut abschirmbar und stellen die Hauptgefahrenquellen für eine ordnungsgemäße Funktion des Implantates dar.

Weitere Abhilfe hat in diesem Zusammenhang die Verwendung von bipolaren Zuleitungen und Elektroden gebracht, die das Niveau von Interferenzen zumindest abschwächen. Jedoch können auch mit solchen Typen von Signalleitungen die Interferenzen nicht völlig eliminiert werden.

Schließlich ist es aus dem Stand der Technik, wie z.B. der US-A-5 292 348, der US-A-5 817 134 oder der US-A-5 857 977, bekannt, durch Detektion des Zeitpunktes des Auftretens von Signalen an zwei Elektroden-Polen unterschiedliche Tachykardien zu erkennen und zu unterscheiden. Dabei werden aufwendige Auswerte-Algorithmen, wie das Kreuz-Phasen-Spektrum oder die Kreuz-Korrelation der Signale eingesetzt.

Ausgehend von der geschilderten Problematik zum Stand der Technik liegt der Erfindung die Aufgabe zugrunde, ein besonders einfach zu implementierendes Verfahren zur Unterscheidung von Interferenzen zwischen EMI-Störsignalen und eigentlichen Abtastsignalen z.B. von kardiologischen Implantaten bereitzustellen.

Zur Lösung dieser Aufgabe ist erfindungsgemäß ein Verfahren zur Unterscheidung zwischen extern eingestrahlten EMI-Störsignalen und elektromedizinischen Abtastsignalen, insbesondere bei Eingangssignalen von kardiologischen Implantaten, mit folgenden Verfahrensschritten vorgesehen:
- Vorverstärkung der Abtastsignale mittels zweier Vorverstärker,
- Vergleichen der vorverstärkten Abtastsignale mittels eines Komparators,
- phasensensitives Auswerten des Komparator-Ausgangssignals zur Differenzierung zwischen Abtastsignalen und EMI-Störsignalen,
- Rückkoppelung des Komparator-Ausgangssignals auf eine Verstärkungssteuerung der Vorverstärker derart, daß bei Detektion eines Störsignals das Komparator-Ausgangssignal minimiert wird.

Der Erfindung liegt die Erkenntnis zugrunde, daß es sich bei den EMI-Störsignalen insbesondere bezüglich bei kardiologischen Implantaten verwendeten Bipolar-Abtastelektroden um ein Fernfeld-Signal handelt, dessen Größe vom Abstand der beiden Elektroden abhängig ist. In nachrichtentechnischer Terminologie kann dieser Abstand als "Dipol-Abstand" der Elektroden verstanden werden.

Im Gegensatz zu den EMI-Störsignalen handelt es sich bei dem Abtastsignal, also z.B. bei dem von der bipolaren Elektrode eines Herzschrittmachers erfaßten kardiologischen Signal um ein Nahfeld-Signal, das vom Dipol-Abstand nicht abhängig ist. Das kardiologische Nahfeldsignal beispielsweise stammt von der elektrischen Erregungsausbreitung des Herzens, die sukzessive an den bipolaren Elektroden vorbeiwandert. Dabei werden die beiden Elektroden der Bipolaranordnung zu einem bestimmten Zeitpunkt mit unterschiedlichen Potentialen beaufschlagt. Die Nahfeldsignale an den beiden Elektroden sind also nicht in Phase.

Den oben erörterten Unterschied zwischen Nah- und Fernfeld nützt die Erfindung aus. EMI-Störsignale von einer bestimmten Quelle treten z.B. an den bipolaren Elektroden eines Herzschrittmachers als zwei in Phase befindliche Signale unterschiedlicher Amplitude auf. Dieser Amplitudenunterschied erzeugt tatsächlich das Störsignal, das von dem Abtast- und Auswerte-Schaltkreis eine Implantates als Störung registriert wird.

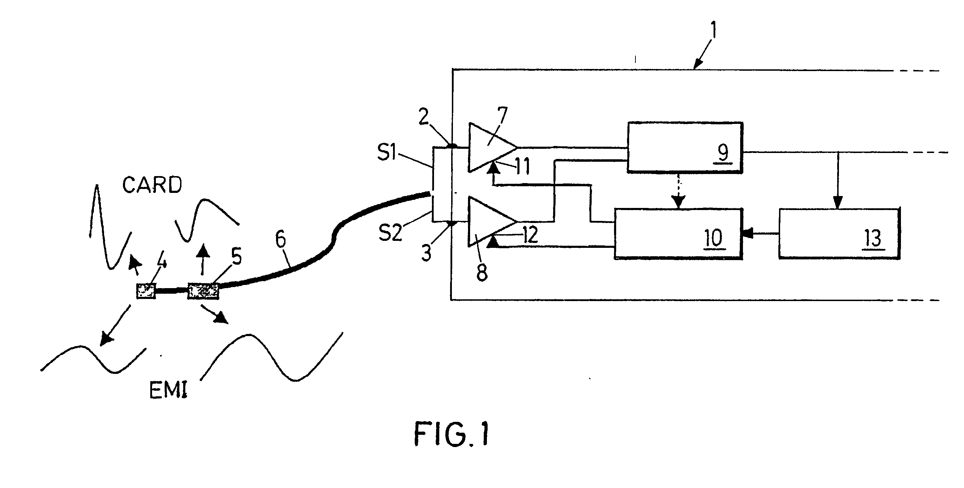

Um diese zu eliminieren ist das erfindungsgemäße Verfahren und die entsprechende Vorrichtung vorgesehen, wie sie in der folgenden Beschreibung eines Ausführungsbeispieles anhand der beigefügten Zeichnung näher erläutert werden. Diese
- Fig.1: ist ein höchst schematisches Diagramm einer Vorrichtung zur Unterscheidung zwischen extern eingestrahlten elektromagnetischen Störsignalen und bipolaren Abtastsignalen bei Eingangssignalen von kardiologischen Implantaten.

In der Zeichnung ist mit 1 ein Teil eines Defibrillators bezeichnet, in den eingangsseitig eine im folgenden näher zu erläuternde Differenzier- oder Unterscheidungsschaltung vorgesehen ist. Diese ist beiden Eingängen 2, 3 nachgeschaltet, an denen die Signalleitungen S1, S2 der beiden Elektroden 4, 5 einer bipolaren Herzkatheterelektrode 6 angeschlossen sind.

Den Eingängen 2, 3 sind jeweils Operationsverstärker 7, 8 nachgeschaltet, die die über die Eingänge 2, 3 zugeleiteten Eingangssignale CARD bzw. EMI einer Meßverstärkung unterziehen. Die Ausgänge der beiden Vorverstärker 7, 8 sind mit den beiden Eingängen eines Komparators 9 verbunden, der ein Ausgangssignal abgibt, das der Differenz der Pegel an seinen Eingängen entspricht. Dieses Ausgangssignal wird durch eine nachgeschaltete Auswerteschaltung 13 phasensensitiv ausgewertet. Dadurch kann zwischen kardiologischen Abtastsignalen CARD und EMI-Störsignalen EMI differenziert werden. Falls nämlich ein EMI-Störsignal - also ein Fernfeld-Signal - detektiert wird, ist das von der Auswerteschaltung 13 zu bewertende Ausgangssignal des Komparators 9 eine Stufenfunktion. Im Falle eines Nahfeld-Signals, bei dem die an den Elektroden 4, 5 anstehenden Signale bipolare kardiologische Signale sind, hat die Auswerteschaltung 13 aufgrund der Außerphasigkeit dieser Nahfeld-Signale ein entsprechend aussehendes Ausgangssignal des Komparators 9 zu bewerten. Zusammenfassend bleibt also für die Auswerteschaltung 13 lediglich die Notwendigkeit, zwischen einer Stufenfunktion und einem typisch kardiologischen Signalverlauf zu differenzieren.

Die Auswerteschaltung ist nun über eine Verstärkungssteuerschaltung 10 mit den Verstärkungssteuereingängen 11, 12 der beiden Vorverstärker 7, 8 gekoppelt. Die Verstärkungssteuerschaltung 10 ist dabei - wie durch einen durchgezogenen Pfeil angedeutet ist - mit der Auswerteschaltung 13 gekoppelt, so daß im Falle der Detektion eines Störsignals die Vorverstärker 7, 8 durch entsprechende Steuersignale so angesteuert werden, daß das Komparator-Ausgangssignal minimiert wird. Letzteres kann - wie durch den strichlierten Pfeil zwischen den Komparator 9 und der Verstärkungssteuerschaltung 10 angedeutet ist - direkt vom Komparator 9 zur Verstärkungssteuerung 10 durchgeschleift werden.

Die Veränderung der Vorverstärkung an den Vorverstärkern 7, 8 ist für die Funktion des Implantates im übrigen dahingehend relevant, als die dadurch beeinflußte Amplitude der kardiologischen Abtastsignale in ihrem Wert für bestimmte Anwendungen des Implantates aussagekräftig ist. Beispielsweise ist der Amplitudenwert im Hinblick auf Schwellwert-Vergleiche zur Detektion von Herz-Kontraktionen bedeutsam. Durch die erfindungsgemäße Veränderung der Eingangsverstärkung werden zwar einerseits, wie beabsichtigt, Störsignale minimiert, andererseits wird aber auch gerade die Amplitude der Abtastsignale beeinflußt. Um hier eine Kompensation herbeizuführen, kann die Änderung der Vorverstärkung durch Anpassung der Weiterverarbeitung des Komparator-Ausgangssignals kompensiert werden. Bipolare kardiologische Abtastsignale ergeben sich dabei als Ausgangssignal des Komparators. Bei einer Änderung der Eingangsverstärkung zur Eliminierung von Störsignalen wird das Ausgangssignal des Komparators um die betragsmäßige Amplitude der Störsignale verändert. Dies kann bei der nachfolgenden Signal-Verarbeitung berücksichtigt werden, indem beispielsweise das Ausgangssignal des Komparators einer entsprechend variierten Verstärkung oder A/D-Wandlung unterworfen wird.

Unipolare Signale ergeben sich durch Komparation der Eingangssignale einer Elektrode und eines indifferenten Pols, bei dem es sich in der Regel um das Implantat-Gehäuse handelt. Bei der Kompensation kann analog den oben stehenden Ausführungen zur bipolaren Abtastung verfahren werden.

Durch das erfindungsgemäße Verfahren und die entsprechende Vorrichtung können zusammenfassend kardiologische Signale auch in der Anwesenheit größerer EMI-Störsignale detektiert werden. Diese Störsignale stellen also kein Problem mehr für kardiologische Implantate dar. Die Erfindung ermöglicht es ferner, EMI-Störsignale zu erkennen und dann unmittelbar aus bipolaren Abtastsignalen zu eliminieren. Nach einer erfindungsgemäßen Detektion von EMI-Signalen können auch beliebige andere, beispielsweise unipolare Abtastsignale von EMI-Störsignalen, durch eine geeignete Schaltung eliminiert werden.

## Patentansprüche

1. Verfahren zur Unterscheidung zwischen extern eingestrahlten elektromagnetischen Störsignalen und elektromedizinischen Abtastsignalen, insbesondere bei Eingangssignalen von kardiologischen Implantaten, wie Herzschrittmachern, Defibrillatoren und dergleichen, bei neurologischen Reizdetektoren usw., mit folgenden Schritten:
- Vorverstärkung der Abtastsignale mittels zweier Vorverstärker (7, 8),
- Vergleichen der vorverstärkten Abtastsignale mittels eines Komparators (9),
- phasensensitives Auswerten des Komparator-Ausgangssignals zur Differenzierung zwischen Abtastsignalen (CARD) und Störsignalen (EMI), und
- Rückkoppelung des Komparator-Ausgangssignals auf die Verstärkungssteuerung der Vorverstärker (7, 8) derart, daß bei Detektion eines Störsignals das Komparator-Ausgangssignal minimiert wird.

2. Verfahren nach Anspruch 1, **dadurch gekennzeichnet, daß** zur Selektierung von Störsignalen das Komparator-Ausgangssignal auf auftretende Stufenfunktions-Signale detektiert wird.

3. Verfahren nach Anspruch 1 oder 2, **dadurch gekennzeichnet, daß** bei Detektion eines Störsignals und entsprechender Änderung der Vorverstärkung an den Vorverstärkern (7, 8) diese Änderung durch Anpassung der Weiterverarbeitung des Komparator-Ausgangssignals kompensiert wird.

4. Vorrichtung zur Durchführung des Verfahrens nach einem der Ansprüche 1 bis 3, mit
- jeweils einem Vorverstärker (7, 8) für die Abtastsignale (CARD),
- einem mit seinen Eingängen an die Ausgänge der Vorverstärker (7, 8) geschalteten Komparator (9),
- einer zwischen den Ausgang des Komparators und die Verstärkungssteuereingänge 11, 12) der Vorverstärker (7, 8) geschaltete Verstärkungssteuerschaltung (10), und
- einer der Verstärkungssteuerschaltung (10) vorgeschaltete Auswerteschaltung (13) zur phasensensitiven Auswertung des Komparator-Ausgangssignals zur Differenzierung zwischen Abtastsignalen (CARD) und Störsignalen (EMI).

## Claims

1. A method for differentiating between externally irradiated electromagnetic interference signals and electromedical sampling signals, in particular in input signals of cardiological implants, such as cardiac pacemakers, defibrillators, and the like, in neurological stimulus detectors, etc., having the following steps:
- preamplification of the sampling signals using two preamplifiers (7, 8),
- comparing the preamplified sampling signals using a comparator (9),
- phase-sensitive analysis of the comparator output signal to differentiate between sampling signals (CARD) and interference signals (EMI), and
- feeding back the comparator output signal to the preamplification controller of the preamplifiers (7, 8) in such way that if an interference signal is detected, the comparator output signal is minimized.

2. The method according to Claim 1, **characterized in that** stepped function signals occurring in the comparator output signal are detected to select interference signals.

3. The method according to Claim 1 or 2, **characterized in that**, if an interference signal is detected and the preamplification at the preamplifiers (7, 8) is changed accordingly, this change is compensated for by adapting the further processing of the comparator output signal.

4. A device for performing the method according to one of Claim 1 through 3, having
- a preamplifier (7, 8) in each case for the sampling signals (CARD),
- a comparator (9), whose inputs are connected to the outputs of the preamplifiers (7, 8),
- an amplification control circuit (10) connected between the output of the comparator and the amplification control inputs (11, 12) of the preamplifiers (7, 8), and
- an analysis circuit (13), connected downstream from the amplification control circuit (10), for the phase-sensitive analysis of the comparator output signal to differentiate between sampling signals (CARD) and interference signals (EMI).

## Revendications

1. Procédé pour la différenciation entre des signaux parasites électromagnétiques arrivant au plan externe et des signaux de balayage électromédicaux, en particulier en cas de signaux d'entrée d'implants cardiologiques, comme des pacemakers, des défibrillateurs et similaires, en cas de détecteurs d'irritation neurologique, etc., comprenant les étapes suivantes :
- préamplification des signaux de balayage moyen de deux préamplificateurs (7, 8),
- comparaison des signaux de balayage préamplifiés au moyen d'un comparateur (9),
- analyse sensible en phase du signal de sortie du comparateur pour la différentiation entre des signaux de balayage (CARD) et des signaux parasites (EMI), et
- réinjection du signal de sortie du comparateur sur la commande de préamplification des préamplificateurs (7, 8) de sorte que, en cas de détection d'un signal parasite, le signal de sortie de comparateur est minimisé.

2. Procédé selon la revendication 1, **caractérisé en ce que**, pour la sélection de signaux parasites, le signal de sortie du comparateur est détecté au niveau des signaux de fonction discontinue qui apparaissent.

3. Procédé selon la revendication 1 ou 2 **caractérisé en ce que**, en cas de détection de signal parasite et d'une modification correspondante de la préamplification sur les préamplificateurs (7, 8), cette modification est compensée par l'adaptation du traitement ultérieur du signal de sortie du comparateur.

4. Dispositif pour la mise en oeuvre du procédé selon l'une quelconque des revendications 1 à 3, comprenant
- à chaque fois un préamplificateur (7, 8) pour les signaux de balayage (CARD),
- un comparateur (9) commuté avec ses entrées sur les sorties des préamplificateurs (7, 8),
- un circuit de commande d'amplification (10) commuté entre la sortie du comparateur et les entrées de commande d'amplification (11, 12) des préamplificateurs et
- un circuit d'analyse (13) placé en amont du circuit de commande d'amplification (10) pour l'analyse sensible en phase du signal de sortie du comparateur pour la différenciation entre des signaux de balayage (CARD) et des signaux parasites (EMI).
